Europäisches Patentamt

European Patent Office

Office européen des brevets

(19)

(11) Publication number: **0 233 848**
A2

(12) ## EUROPEAN PATENT APPLICATION

(21) Application number: 87830061.5

(22) Date of filing: 20.02.87

(51) Int. Cl.³: **A 61 M 1/18**

(30) Priority: 20.02.86 IT 1948886

(43) Date of publication of application:
26.08.87 Bulletin 87/35

(84) Designated Contracting States:
DE ES FR SE

(71) Applicant: Civati, Giovanni
Via Cardinal Ferrari 60
I-20099 Sesto S. Giovanni (Milano)(IT)

(71) Applicant: Guastoni, Carlo
Via Spartaco 2
I-20135 Milano(IT)

(71) Applicant: Teatini, Ugo
Via C. A. Pisani Dossi 44
I-20134 Milano(IT)

(72) Inventor: Civati, Giovanni
Via Cardinal Ferrari 60,
I-20099 Sesto S. Giovanni (Milano)(IT)

(74) Representative: Bosotti, Luciano et al,
c/o Jacobacci-Casetta & Perani S.p.A. Via Alfieri, 17
I-10121 Torino(IT)

(54) An improved filtering device, particularly for treating kidney insufficiencies.

(57) Apparatus (1) for haemofiltration without replacement liquid is constituted by a single casing comprising separate parts (2, 3) each constituted by a central chamber (6, 9) and an outer jacket (7, 10), the jacket (7) of the first part (2) being in fluid communication (8a) with the chamber (9) of the second part. The blood is introduced into the first chamber (6) from which it leaves, purified and concentrated, through a tube (15), while the filtrate collected in the first jacket (7) passes into the second chamber (9) in correspondence of which it comes into contact with a dialysis liquid circulated (11, 17) through the second jacket (10). The purified filtrate is then taken (12) from the second chamber (9) and recombined with the concentrated blood coming from the first chamber (15) for reintroduction into the patient's body.

EP 0 233 848 A2

An improved filtering device, particularly for treating
renal failure.

The present invention relates to apparatus for purifying
the blood   of  uraemic patients  and  particularly  to
integrated    apparatus   for    haemofiltration    without
replacement liquid.

It   is   known that uraemic patients, for  example, as  a
result  of acute renal failure or  particularly  chronic
renal  failure, must undergo periodic blood-purification
treatment in order to remove the  excess of  nitrogenous
by-products  resulting from protein  metabolism,  which,
if allowed to accumulate to excess, would lead to  death
through   a   succession   of   progressively   more  serious
morbid conditions, such as encephalopathy and coma.

The  purification of the blood of uraemic  patients  has
been   carried   out   for   some   time   by   means   of
haemo-dialysis  based on the physical principle of  the
diffusion of solutes through semi-permeable membranes in
proportion to the concentration gradients present on the
two  sides  of  the membranes.   In  the  haemo-dialysis
process,  the  blood is brought into contact  through  a
suitable  membrane   with   a   dialysis liquid having a
composition  and  concentration  arranged  so  that  the
nitrogenous  toxins  can  pass from the  blood  to  the
dialysis liquid and be removed therewith.

Another  blood-purification   system   which  came   into
practice  only recently is haemofiltration based on  the
forced  passage  of the plasma liquid  and  its  solutes
through  artificial semi-permeable membranes  under  the
action   of   a   suitable   pressure.    Since,    in
haemofiltration,  the  blood  undergoes  a  considerable
reduction  in  volume  as a result  of  loss  of  plasma

- 2 -

liquid, it is necessary to restore the original volume by the introduction of a suitable reinfusion fluid of predetermined composition and concentration.

The advantages of haemofiltration over dialysis are constituted by a more harmonic purification over a wide molecular spectrum since, while the passage of the solutes in dialytic diffusion is inversely proportional to their mass, in haemofiltration the forced passage is theoretically equal for all those solutes whose mass is less than the exclusion limit (cut-off point) of the membrane; this gives a better physiological character in respecting the basic equilibrium of the sodium and the bicarbonates in the body and better stability and cardiovascular tolerability to weight loss. On the other hand, the disadvantages of haemofiltration are constituted essentially by the relative complexity of the necessary apparatus and the cost of the reinfusion fluid for replacing the lost portion of the plasma, costs linked mainly to the requirement for the provision of fluids which are absolutely free from undesirable and pyrogenic substances.

Another blood-purification technique which has been under clinical experimentation even more recently is so-called haemofiltration without replacement fluid, which attempts to eliminate the replacement fluid by re-using the same plasma ultrafiltrate from the patient after regeneration by a dialytic process. In this system, the purification occurs, in the final analysis, in an even more diffuse way, it being not the blood which is in contact with the semi-permeable membrane in the "haematic" compartment but pure plasma ultrafiltrate which has an aqueous consistency and hence little viscosity, and is free from cells, proteins and

- 3 -

so-called "reactants" of the acute phase. This peculiarity, constituted by the physical separation of the blood from the dialysis liquid, enables the dialytic diffusion to be carried out under particularly advantageous conditions, the haemodynamic advantages being maintained and improved and the equilibrium of the electrolytes and equally the acid-base equilibrium being respected. This has been ascertained in the course of numerous tests with various patients, which have confirmed the innocuous nature and validity of this therapeutic approach.

A technical disadvantage of this haemofiltration process is the need encountered until now of having to have recourse to the skilled assembly of numerous elements available commercially for other purposes, such as the haemofilter, the regenerating filter, the tubes or lines which connect the various elements, etc.

The main object of the present invention is to provide simple and reliable apparatus which enables haemodialysis without liquid replacement to be carried out simply and safely, minimising the connection points and hence the possibility of liquid losses, contamination and infection.

The integrated haemofiltration apparatus according to the present invention is constituted by a single body divided into two sections, of which that intended to receive the arriving blood comprises a central portion constituted by a series of capillaries and an outer jacket intended to receive the liquid transuded by the semi-permeable membranes of the capillaries while the other section, which has a structure and conformation substantially like the previous one, comprises a central network of capillaries intended to receive the ultrafiltration liquid coming from the jacket of the first section and an outer jacket in which a suitable dialysis liquid circulates. The purified plasma fluid ultrafiltrate from the central jacket of the second section is withdrawn by a peristaltic pump and recombined with the concentrated blood from the central ultrafiltration chamber of the first section, and the blood thus reintegrated is passed back into the patient's body.

The apparatus of the present invention will now be explained in more detail with reference to a possible embodiment shown schematically in the single appended drawing.

The apparatus according to the present invention, generally indicated 1, is constituted essentially by an elongate body, preferably of cylindrical form, which is divided by a central partition into two portions of which the first, indicated 2, is adapted to receive the pressurised blood flow coming from the patient's body and may be termed the filtration section, while the second portion, indicated 3, wherein the liquid plasma obtained by filtration in the first section is handled may be termed the dialysis section. The blood taken from the patient and pressurised by a peristaltic pump 5 enters the filtration section 2 through a hole formed in the closure plate 4; the pressurised blood enters the central chamber 6 which comprises a network of semi-permeable capillaries, and the fluid plasma containing the nitrogenous waste and the electrolytes which pass through the barrier constituted by the membranes of the capillaries collects in a surrounding jacket 7. The two sections 2 and 3 of the apparatus are separated by a wall 8 within which are one or more suitably cross-sectioned passages 8a that put the outer jacket 7 in which the filtered plasma liquid collects into communication with the central chamber 9 of the dialysis section 3. The chamber 9 in turn is constituted by a network of capillaries surrounded by an outer jacket 10 in which a dialysis liquid coming from a tube 11 circulates. A tube 12 puts the central chamber 9 of the dialysis section 3 into communication with a peristaltic pump 13 which draws in the liquid purified in the dialysis process and forces it into a manifold 14 where the purified ultrafiltrate mixes with the concentrated blood coming through a tube 15 from the central chamber 6 of the filtration section 2.

A blood flow of between 400 and 600 ml/min is taken by the peristaltic pump 5 through an external shunt or artero-venousfistula of the patient and conveyed along the tube 16 to the central chamber 6 of the filtration section 2 constituted by the network of semi-permeable capillaries. By virtue of the hydrostatic pressure which is exerted on the walls of the capillaries, the plasma and the solids contained therein leave at a predetermined flow (ultrafiltration duty) and collect in the surrounding jacket 7, progressively expelling the air initially contained therein until it occupies the entire volume. The plasma liquid thus obtained then passes through the passages 8a formed in the partition wall 8 and reaches the central chamber 9 of the dialysis section 3 itself constituted by capillaries with semi-permeable walls which are, however, in contact with a flow of dialysis liquid of known composition which flows from the tube 11 at a rate equal to or greater than 500

- 6 -

ml/min. In this second part of the apparatus the ultrafiltrate constituted by the plasma liquid from the jacket 7 loses the greater part of the toxins typical of uraemic plasma and is equilibrated simultaneously by dialysis with its electrolytic solutes. The plasma ultrafiltrate thus regenerated and purified is withdrawn by the peristaltic pump 13, mixed in the manifold 14 with the blood flowing from the capillary chamber 6 of the filtration section 2 along the tube 15, and the re-integrated and reconstituted blood returned to the patient through the tube 18. In its turn, the dialysis fluid loaded with waste products circulating in the jacket 10 flows along the tube 17 along which is located a system for controlling the final volume loss.

The advantage of the use of a dialysis liquid instead of the reinfusion fluid provided in normal haemofiltration lies in the fact that it is cheaper and does not come in direct contact with the blood, thus completely eliminating any possibility of contamination.

In their turn, the flows produced by the peristaltic pumps 5 and 13 must be commensurate with each other in order to avoid imbalances in the flows on one side and the other. More particularly, the flow rate of the suction pump 13 should be at least about 40% less than that of the pressurising perastaltic pump 5.

The integrated haemofiltration apparatus without fluid replacement according to the present invention enables the detoxification of uraemic patients to be simplified and rationalised with considerable efficiency with respect to physiological parameters and exclusion of undesirable side effects. The compact structure of the apparatus described, as well as simplifying the execution and carrying out of the haemofiltration, also enables the overall costs to be reduced while preserving and increasing the advantageous physiological characteristics of haemofiltration over haemodialysis.

Although the present invention has been described and illustrated on the basis of a specific embodiment, it is clear that variants and/or modifications may be made by experts in the art without thereby departing from the scope of protection and the spirit of the invention itself. In particular, the overall form of the apparatus could be modified, for example, by the adoption of prismatic shapes rather than cylindrical ones.

- 8 -

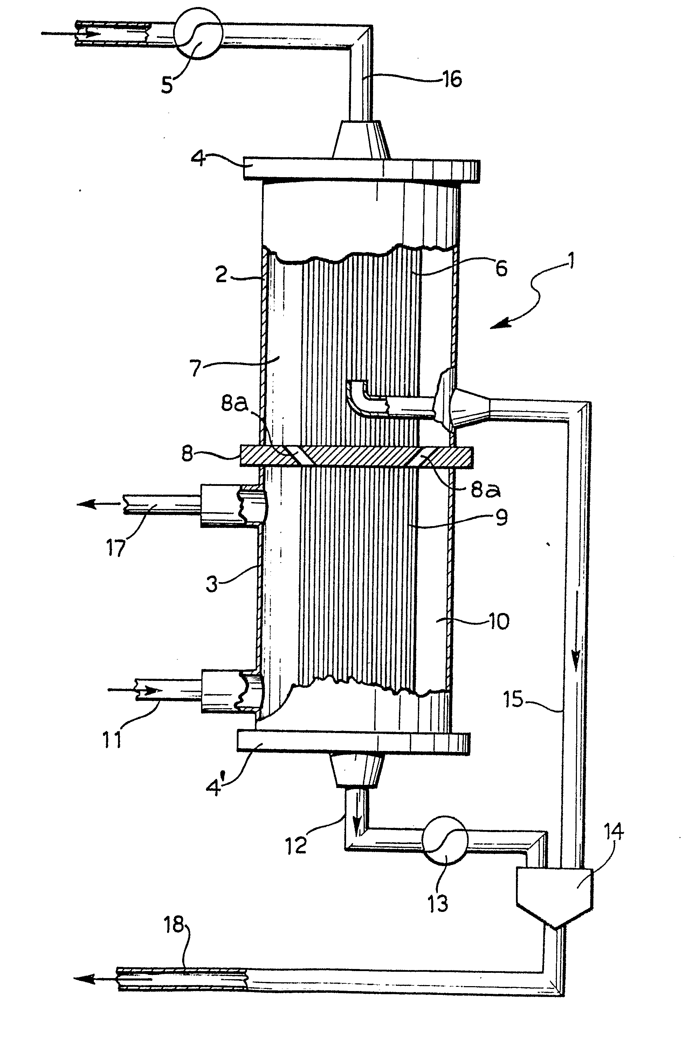

## CLAIMS

1.      Apparatus (1) for haemofiltration without fluid replacement, comprising essentially haemofiltration apparatus (2) and purification and dialysis apparatus (3) characterised in that it is constituted by a single element (1) formed by a filtration section (2) comprising a central chamber (6) and a surrounding jacket (7) and a dialysis section (3) similarly comprising a central chamber (9) with semi-permeable capilliaries surrounded by a peripheral chamber (10), the two filtration (2) and dialysis (3) sections being separated from each other by a partition wall (8) within which are formed communication passages (8a) between the jacket (7) of the filtration section (2) and the central chamber (9) of the dialysis section (3).

2.      Haemofiltration apparatus according to Claim 1, characterised in that the filtration (2) and dialysis (3) sections separated by the partition wall (8) are closed at respective ends by closure plates (4,4') having respective apertures for connection to an inlet tube (16) and an outlet tube (12), the central chamber (6) with the semi-permeable capillaries of the filtration section (2) being connectible directly to an outflow tube (15) and the outer jacket (10) of the dialysis section (3) being connectible to aninlet tube (11) and an outlet tube (17) for the circulation of the dialysis liquid.

0233848